# EUROPEAN PATENT APPLICATION

(11) **EP 4 796 565 A1**
(43) Date of publication of application: **26.08.2026**
(21) Application number: 24879126.1
(22) Date of filing: 18.10.2024
(51) Int. Cl.: C07K 19/00, C12N 9/12, A61K 38/16, A61P 35/00

(54) **SYNTHESIZED ANTI-CANCER LIPOPEPTIDE AND USE THEREOF**

(30) Priority: 18.10.2023 CN 202311352929
(71) Applicant: Yongzhou Zhonggu Biotechnology Co., Ltd., Hunan 425000 (CN); Centro De Ingeniería Genética Y Biotecnología (CIGB), Playa, Ciudad De La Habana 10 600 (CU)
(72) Inventor: PEREA RODRIGUEZ, Silvio E., Ciudad De La Habana 10600 (CU); PERERA NEGRIN, Yasser, Ciudad De La Habana 10600 (CU); DAI, Lingfeng, Hunan 425000 (CN); YI, Ying, Hunan 425000 (CN); REYES ACOSTA, Osvaldo, Ciudad De La Habana 10600 (CU); GARAY, Hilda E., Ciudad De La Habana 10600 (CU); GONZALEZ BLANCO, Sonia, Ciudad De La Habana 10600 (CU); TAN, Changyuan, Hunan 425000 (CN); YANG, Ke, Hunan 425000 (CN)
(74) Representative: Colombo, Stefano Paolo
(86) International application number: PCT/CN2024/125799
(87) International publication number: WO 2025/082486

(57) **Abstract**

Provided are a synthesized anti-cancer lipopeptide and the use thereof. Specifically, the anti-cancer lipopeptide comprises a domain X₁, X₂ and X₃, wherein: X₁ is selected from C₆-C₂₀ fatty acid; X₂ is selected from fragments of cell-penetrating peptides (CPPs); and X₃ is selected from fragments of peptides that specifically bind to protein kinase 2 (CK2). Compared with CIGB-300, the polypeptide can more effectively inhibit CK2, has a higher RPS6 protein-targeting property, has better tumor inhibitory activity, anti-metastatic effect and safety, and can enable large-scale production.

## Description

The present application is based on and claims priority to the application with application number CN 202311352929.6 and application date October 18, 2023, the content of which is hereby incorporated by reference in its entirety.

### Technical Field

The present application relates to the field of cancer treatment, specifically to a synthetic anti-cancer lipopeptide and use thereof.

### Prior Art

Protein kinase CK2 has become an important molecular target in the field of protein kinases, playing a crucial role in cancer cell biology (Buontempo et al, 2018; Aasebø et al, 2020; Klink et al, 2021). Increasing evidence suggests that CK2 plays an important role in human malignant tumor research.

Preclinical studies have explored various strategies for inhibiting CK2 activity. However, only two compounds, the ATP-competitive inhibitor CX-4945 and the CIGB-300 peptide, have entered clinical trials in cancer patients as anti-CK2 drugs (Zakharia et al, 2019; Perea et al, 2018). The CIGB-300 peptide is a peptide chimera that comprises a cyclic peptide inhibiting CK2-mediated phosphorylation and a cell-penetrating peptide, Tat, that facilitates its delivery into cells. It is a "first-in-class" synthetic peptide that has entered clinical trials, inhibiting CK2-mediated phosphorylation through a dual mechanism targeting both the substrate phosphoacceptor domain and the CK2α subunit (Perea et al, 2004; Perera et al, 2020). Proteomic analysis of the CIGB-300 peptide in non-small cell lung cancer (NSCLC) cells revealed that the CIGB-300 peptide modulates various proteins involved in apoptosis, cell proliferation, ribosome biogenesis, anti-cancer drug resistance, cell metastasis and angiogenesis (Rodriguez-Ulloa et al, 2010). Similarly, phosphoproteomic studies investigating the effects of the CIGB-300 peptide in NSCLC cells identified a set of CK2 substrates that were significantly suppressed alongside disruptions in cellular processes (Perera et al, 2020). Therefore, the CIGB-300 peptide not only induces apoptosis and inhibits cell proliferation but also exerts anti-angiogenic/anti-metastatic effects in mouse cancer models (Benavent et al, 2011; Benavent et al, 2016). The CIGB-300 peptide exhibits synergistic effects when combined with paclitaxel, cisplatin, cytarabine (AraC), and epidermal growth factor receptor (EGFR) inhibitors such as erlotinib and nimotuzumab (Perea et al, 2015; Perea et al, 2018). The antitumor activity of the CIGB-300 peptide has been validated in mouse cancer models via both local intratumoral injection (Perea et al, 2004) and systemic administration routes (Perea et al, 2008). Currently, the CIGB-300 peptide is under clinical investigation, with its safety, tolerability, pharmacokinetics, and efficacy data confirmed in Phase I and II clinical trials involving patients with tumors of various origins (Soriano et al, 2013; Sarduy et al, 2015; Batista et al, 2018).

However, some tumor cells exhibit intrinsic resistance to the anti-proliferative and pro-apoptotic effects of the CIGB-300 peptide, possibly due to the rate of peptide internalization and nucleolar localization (Perera et al, 2012; Benavent et al, 2014).

### Contents of the Invention

The present invention describes a synthetic anti-cancer lipopeptide (SEQ ID NO: 1, referred to as "lipopeptide"), which exhibits improved anti-tumor effects compared to the CIGB-300 peptide, with a 4-to 11-fold increase in sensitivity to tumor cells. Furthermore, the lipopeptide of the present invention has low manufacturing costs and can be produced on a large scale.

Specifically, in a first aspect, the present application provides a non-natural polypeptide, which comprises domains X₁, X₂, and X₃, wherein:
X₁ is selected from C₆-C₂₀ fatty acid residues;
X₂ is selected from fragments of a cell-penetrating peptide (CPP);
X₃ is selected from fragments of peptides capable of specifically binding to protein kinase 2 (CK2).

In a second aspect, the present application provides a pharmaceutical composition, which comprises the non-natural polypeptide as described in any one of the items of the first aspect and a pharmaceutically acceptable excipient.

In a third aspect, the present application provides a kit, which comprises the non-natural polypeptide as described in any one of the items of the first aspect or the pharmaceutical composition as described in any one of the items of the second aspect, and optionally a second therapeutic agent.

In another aspect, the present application provides a use of the non-natural polypeptide as described in any one of the items of the first aspect, the pharmaceutical composition as described in any one of the items of the second aspect, or the kit as described in any one of the items of the third aspect in the manufacture of a CK2 modulator, or in the manufacture of a medicament, wherein the medicament is used for:
(1) inhibiting proliferation or metastasis of a tumor cell, or promoting apoptosis of the tumor cell;
(2) inhibiting angiogenesis, tumor growth, or metastasis;
(3) treating a disease associated with CK2 activity.

In another aspect, the present application provides a method for modulating CK2 activity in a cell, wherein the method comprises contacting the cell with the non-natural polypeptide as described in any one of the items of the first aspect, the pharmaceutical composition as described in any one of the items of the second aspect, or the kit as described in any one of the items of the third aspect.

In another aspect, the present application provides a method for inhibiting proliferation or metastasis of a tumor cell, or promoting apoptosis of the tumor cell, wherein the method comprises administering to a subject an effective amount of the non-natural polypeptide as described in any one of the items of the first aspect, the pharmaceutical composition as described in any one of the items of the second aspect, or the kit as described in any one of the items of the third aspect.

In another aspect, the present application provides a method for inhibiting angiogenesis, or inhibiting tumor growth or metastasis, wherein the method comprises administering to the subject an effective amount of the non-natural polypeptide as described in any one of the items of the first aspect, the pharmaceutical composition as described in any one of the items of the second aspect, or the kit as described in any one of the items of the third aspect.

In another aspect, the present application provides a method for treating a disease associated with CK2 activity in a subject, wherein the method comprises administering to the subject a therapeutically effective amount of the non-natural polypeptide as described in any one of the items of the first aspect, the pharmaceutical composition as described in any one of the items of the second aspect, or the kit as described in any one of the items of the third aspect.

### Brief Description of the Drawings

The accompanying drawings are provided to further illustrate the present invention and form a part of the present invention. The illustrative examples and descriptions of the present invention are intended to explain the present invention and shall not constitute an undue limitation thereof. In the drawings:
Figure 1 shows an MS spectrum of the lipopeptide.
Figure 2A shows the apoptosis induced by the lipopeptide and the CIGB-300 peptide in NCI-H226 cells, quantified by flow cytometry following annexin V staining.
Figure 2B shows the apoptosis induced by the lipopeptide and CIGB-300 peptide in SK-MES1 cells, quantified by flow cytometry following annexin V staining.
Figure 3 shows the in vivo toxicity of the lipopeptide and the CIGB-300 peptide, with animal body weight recorded as a marker of therapeutic toxicity.
Figure 4 shows the inhibitory effects of the lipopeptide and the CIGB-300 peptide on phosphorylation and CK2-related proteins such as cdc37 in the NCI-H226 cell line, analyzed by Western blotting experiments.
Figure 5 shows the specific binding of the lipopeptide and the CIGB-300 peptide to RPS6 protein in the NCI-H226 cell line, which was analyzed by Western blotting and further quantified by densitometry.

### Definitions of terms

As used herein, unless otherwise specified, the scientific and technical terms used have the meanings commonly understood by those skilled in the art. Furthermore, the procedures involving molecular genetics, nucleic acid chemistry, cell culture, biochemistry, cell biology, and other related techniques described herein are conventional methods widely used in their respective fields. Meanwhile, to facilitate a better understanding of the present invention, definitions and explanations of relevant terms are provided below.

As used herein, the singular forms "a", "an", and "the" are intended to include the plural forms as well, unless the context clearly indicates otherwise. Furthermore, the terms "comprising", "including", "having", "containing", or variations thereof are open-ended and are not exclusive or exhaustive. Moreover, the terms "herein", "above", "below", or words of similar meaning shall refer to the present application as a whole and not to any particular part thereof.

As used herein, the amino acid residue abbreviations are as follows: alanine (Ala; A), asparagine (Asn; N), aspartic acid (Asp; D), arginine (Arg; R), cysteine (Cys; C), glutamic acid (Glu; E), glutamine (Gln; Q), glycine (Gly; G), histidine (His; H), isoleucine (Ile; I), leucine (Leu; L), lysine (Lys; K), methionine (Met; M), phenylalanine (Phe; F), proline (Pro; P), serine (Ser; S), threonine (Thr; T), tryptophan (Trp; W), tyrosine (Tyr; Y), and valine (Val; V). In some embodiments, the amino acids used may be common amino acids, such as the 20 kinds of protein L-amino acids. In some embodiments, the amino acids used may be non-natural amino acids. The term "non-natural amino acids" as used herein may include any amino acid other than the 20 kinds of proteinogenic L-amino acids. These amino acids may include amino acids with non-classical side chains, D-amino acids, β-amino acids, etc. Unless otherwise specified, amino acids used herein are of the L-configuration.

As used herein, the terms "subject", "patient", or "individual" include mammals and non-mammals. Mammals may be any member of the class Mammalia, including but not limited to humans; non-human primates such as chimpanzees, apes, or other monkeys; livestock such as cattle, horses, sheep, goats, and pigs; domestic animals such as rabbits, dogs (or canines), and cats; laboratory animals including rodents such as rats, mice, and guinea pigs; and the like. Non-mammals may include birds, fish, and the like. In some embodiments, the subject may be a mammal. In some embodiments, the subject may be a human. In some embodiments, the human may be an adult. In some embodiments, the human may be a child. In some embodiments, the human may be 0 to 17 years old. In some embodiments, the human may be 18 to 130 years old. In some embodiments, the subject may be male. In some embodiments, the subject may be female. In some embodiments, the subject is diagnosed with or suspected of having a certain disease. In some embodiments, the disease is cancer. The subject may be a patient or an individual. In some embodiments, the terms subject, patient, or individual may be used interchangeably.

As used herein, the terms "treatment", "management", "improvement", or "relief" include alleviating or reducing the symptoms of a disease, inhibiting a disease (e.g., preventing the progression of a disease), alleviating a disease, causing the regression of a disease, alleviating symptoms caused by a disease, or arresting the symptoms of a disease. The terms "treatment", "management", "improvement", or "relief" may further include achieving a therapeutic benefit. Therapeutic benefits may refer to the eradication of the disease being treated. Additionally, therapeutic benefits may also be achieved by eradicating one or more physiological symptoms associated with the disease being treated, such that an observable improvement is attained in the subject, even though the subject may still be afflicted with the underlying disease in some embodiments.

As used herein, the terms "effective amount" and "therapeutically effective amount" refer to a sufficient amount of an administered agent that will, at least in part, alleviate the symptoms of the disease being treated. Dosage regimens may be adjusted to provide the optimal desired response. For example, a single bolus may be administered, several divided doses may be administered over time, or the dose may be proportionally reduced or increased according to treatment progress. It is noted that dosage values may vary with the type and severity of the disease to be alleviated and may include single or multiple doses. It is to be further understood that for any particular individual, specific dosage regimens should be adjusted over time based on individual needs and the drug's instructions or the professional judgment of a clinician. Generally, effective doses range from about 0.0001 to about 50 mg per kg body weight per day, for example, from about 0.01 to about 10 mg/kg/day (administered in single or divided doses). For a 70 kg person, this would amount to from about 0.007 mg/day to about 3500 mg/day, for example, from about 0.7 mg/day to about 700 mg/day. In some embodiments, dosage levels below the lower limit of the aforementioned range may be adequate, while in other cases, larger doses may still be employed without causing any harmful side effects, provided that such larger doses are first divided into several smaller doses for administration throughout the day.

As used herein, the terms "peptide" and "polypeptide" are used interchangeably to cover naturally occurring and non-natural proteins and their fragments, mutants, derivatives, and analogues. Polypeptides may be monomeric or polymeric. Furthermore, a polypeptide may contain multiple distinct domains, each possessing one or more distinct activities. A "polypeptide" may be of any length greater than two amino acids. In some embodiments, a peptide may be positively charged as a whole. In some embodiments, a peptide may be negatively charged as a whole. In some embodiments, a peptide may be neutrally charged as a whole. Peptides may also exist in zwitter ionic form.

The peptides described herein may be in the form of their salts. In some embodiments, references to the term "peptide" or "polypeptide" are to be interpreted as including their salts, even if not explicitly stated. In some embodiments, the salts may include carboxylic acid salts (e.g., formate, acetate, trifluoroacetate, trichloroacetate, propionate, isobutyrate, heptanoate, caprate, caprylate, stearate, acrylate, caproate, propionate, ascorbate, citrate, glucuronate, glutamate, glycolate, α-hydroxybutyrate, lactate, tartrate, phenylacetate, mandelate, phenylpropionate, phenylbutyrate, benzoate, chlorobenzoate, methylbenzoate, hydroxybenzoate, methoxybenzoate, dinitrobenzoate, o-acetoxybenzoate, salicylate, pamoate, nicotinate, isonicotinate, cinnamate, oxalate, malonate, succinate, suberate, sebacate, fumarate, malate, maleate, hydroxymaleate, hippurate, phthalate, or terephthalate); halide salts (e.g., chloride, bromide, or iodide salts); sulfonates (e.g., benzenesulfonate, methyl-, bromo-, or chlorobenzenesulfonate, xylenesulfonate, methanesulfonate, trifluoromethanesulfonate, ethanesulfonate, propanesulfonate, hydroxyethanesulfonate, 1- or 2-naphthalenesulfonate, or 1,5-naphthalenedisulfonate); sulfates; pyrosulfates; bisulfates; sulfites; bisulfites; phosphates; monohydrogen phosphates; dihydrogen phosphates; metaphosphates; pyrophosphates; nitrates; and the like. In some embodiments, the salts may be pharmaceutically acceptable salts. Pharmaceutically acceptable salts may be those described in Berge et al., J. Pharm. Sci., 1977.

As used herein, the term "identity" refers to the sequence matching between two polypeptides or two nucleic acids. When a position in the two sequences being compared is occupied by the same base or amino acid monomer subunit (for example, a position in each of two DNA molecules is occupied by adenine, or a position in each of two polypeptides is occupied by lysine), then the molecules are identical at that position. The "percent identity" between two sequences is a function of the number of matching positions shared by the two sequences divided by the number of positions compared × 100. For example, if 6 out of 10 positions in two sequences match, then the two sequences have an identity of 60%. For instance, the DNA sequences CTGACT and CAGGTT share an identity of 50% (3 positions out of a total of 6 positions match). Typically, comparisons are made by aligning the two sequences to achieve maximum identity. Such alignments may be performed using, for example, the method of Needleman et al. (1970) J. Mol. Biol. 48:443-453, which may be conveniently carried out using computer programs such as the Align program (DNAstar, Inc.). The percent identity between two amino acid sequences can also be determined using the algorithm of E. Meyers and W. Miller (Comput. Appl Biosci., 4:11-17 (1988)) integrated into the ALIGN program (version 2.0), employing the PAM120 weight residue table, a gap length penalty of 12, and a gap penalty of 4. Additionally, the percent identity between two amino acid sequences may be determined using the algorithm of Needleman and Wunsch (J Mol Biol. 48:444-453 (1970)) integrated into the GAP program within the GCG software package (available at www.gcg.com), using a Blossum 62 matrix or a PAM250 matrix, along with gap weights of 16, 14, 12, 10, 8, 6, or 4 and length weights of 1, 2, 3, 4, 5, or 6.

As used herein, the terms "co-administration" and "combined administration" comprise administering a selected therapeutic agent (i.e., the second therapeutic agent described herein) to a subject, and may comprise a treatment regimen in which the agents are administered via the same or different routes of administration or at the same or different times. In some embodiments, the polypeptide described herein may be co-administered with an additional therapeutic agent. This comprises administering two or more agents to a subject such that the agents and/or their metabolites are simultaneously present in the subject, optionally comprising simultaneous administration, administration at different times, and/or administration in a composition where both agents are present. Therefore, in some embodiments, the peptide and the second therapeutic agent may be administered in a single composition. In some embodiments, the peptide and the second therapeutic agent may be mixed in a composition. In some embodiments, the same peptide or therapeutic agent may be administered via a combination of different routes of administration. In some embodiments, each therapeutic agent administered may be in a therapeutically effective amount.

### Anti-cancer lipopeptide

The present application provides a non-natural polypeptide, which comprises a CK2-targeting cyclic peptide and a cell-penetrating peptide, wherein the non-natural polypeptide comprises a lipophilic carboxylic acid modification at the N-terminus. Compared to CIGB-300, the polypeptide can more effectively inhibit CK2, and has higher targeting specificity to RPS6 protein, better tumor suppressor activity and anti-metastatic effect, as well as higher safety.

In some embodiments, the present application provides a non-natural polypeptide, which comprises domains X₁, X₂, and X₃, wherein:
X₁ is selected from C₆-C₂₀ fatty acid residues;
X₂ is selected from fragments of a cell-penetrating peptide (CPP);
X₃ is selected from fragments of peptides capable of specifically binding to protein kinase 2 (CK2).

In some embodiments, X₁ is selected from C₆-C₁₂ fatty acid residues, such as C₆, C₇, C₈, C₉, C₁₀, C₁₁, or C₁₂ fatty acid residues. In some embodiments, X₁ is decanoyl.

In some embodiments, X₂ comprises a fragment of a peptide having an identity of at least 50%, 55%, 60%, 65%, 70%, 75%, 80%, 85%, 90%, 95%, 98%, or 100% as compared with GRKKRRQRRRPPQ (SEQ ID NO: 2).

In some embodiments, X₃ has CK2 inhibitory activity, such as inhibiting CK2 phosphorylation.

In some embodiments, X₃ comprises a fragment of a peptide having an identity of at least 50%, 55%, 60%, 65%, 70%, 75%, 80%, 85%, 90%, 95%, 98%, or 100% as compared with CWMSPRHLGTC (SEQ ID NO: 3, wherein a disulfide bond is formed between cysteines at position 1 and position 11).

In some embodiments, an amino acid or a peptide linker exists between two adjacent domains of X₁, X₂, and X₃.

In some embodiments, X₁, X₂, and X₃ are linked in any order.

In some embodiments, X₁, X₂, and X₃ are arranged sequentially from the N-terminus to the C-terminus.

In some embodiments, the polypeptide is connected from the N-terminus to the C-terminus in the order of X₁-X₂-β-Ala-X₃.

In some embodiments, the C-terminus of the aforementioned non-natural polypeptide is modified by amidation.

In some embodiments, the non-natural polypeptide comprises or is composed of the following amino acid sequence:
X₁-Gly¹-Arg² -Lys³ -Lys⁴ -Arg⁵-Arg⁶-Gln⁷-Arg⁸-Arg⁹-Arg¹⁰-Pro¹¹-Pro¹²-Gln¹³-p-Ala¹⁴-Cys¹⁵-Trp¹⁶-Met¹⁷-Ser¹⁸-Pro¹⁹-Arg²⁰-His²¹-Leu²²-Gly²³-Thr²⁴-Cys²⁵-NH₂ (SEQ ID NO: 1), wherein,
X₁ is a decanoyl group;
a disulfide bond is formed between Cys¹⁵ and Cys²⁵.

In some specific embodiments, the chemical name and structure of the lipopeptide described herein are as follows:
(15→25) (Cyclic disulfide) decanoyl-glycyl-L-histidyl-L-lysyl-L-lysyl-L-arginyl-L-arginyl-L-glutaminyl-L-arginyl-L-arginyl-L-arginyl-L-prolyl-L-prolyl-L-glutaminyl-β-alanyl-L-cysteinyl-L-tryptophyl-L-methionyl-L-seryl-L-prolyl-L-arginyl-L-histidyl-L-leucyl-glycyl-L-threonyl-L-cysteinamide.

### Synthesis of anti-cancer lipopeptide

Those skilled in the art are aware of various suitable methods that may be used to synthesize peptides. In some embodiments, the peptide described herein may be synthesized in liquid-phase system or solid-phase system using techniques known in the art (see, for example, Atherton, E., Sheppard, R.C. (1989). Solid Phase peptide synthesis: a practical approach. Oxford, England: IRL Press; Stewart, J. M., Young, J. D. (1984). Solid phase peptide synthesis (2nd edition). Rockford: Pierce Chemical Company).

### Pharmaceutical composition and kit

In another aspect, the present application provides a pharmaceutical composition, which comprises the non-natural peptide as described in any one of the aforementioned items and a pharmaceutically acceptable excipient.

The excipient may be one described in the Handbook of Pharmaceutical Excipients, American Pharmaceutical Association (1986). Non-limiting examples of suitable excipients include buffers, preservatives, binders, lubricants, disintegrants, chelating agents, surfactants, flavoring agents, sweeteners, and colorants.

In some embodiments, suitable buffers include calcium bicarbonate, sodium bicarbonate, potassium bicarbonate, magnesium hydroxide, magnesium lactate, magnesium gluconate, aluminum hydroxide, sodium citrate, sodium tartrate, sodium acetate, sodium carbonate, sodium polyphosphate, potassium polyphosphate, sodium pyrophosphate, potassium pyrophosphate, disodium hydrogen phosphate, dipotassium hydrogen phosphate, trisodium phosphate, tripotassium phosphate, potassium metaphosphate, magnesium oxide, magnesium hydroxide, magnesium carbonate, magnesium silicate, calcium acetate, calcium glycerophosphate, calcium chloride, calcium hydroxide, and other calcium salts, or combinations thereof.

In some embodiments, suitable preservatives include antioxidants such as α-tocopherol and ascorbic acid salts, and antimicrobial agents such as parabens, chlorobutanol, and phenol. Antioxidants may further include EDTA, citric acid, ascorbic acid, butylated hydroxytoluene (BHT), butylated hydroxyanisole (BHA), sodium sulfite, para-aminobenzoic acid, glutathione, propyl gallate, cysteine, methionine, ethanol, and *N*-acetylcysteine, etc.

In some embodiments, suitable binders include starches such as potato starch, corn starch, and wheat starch; sugars such as sucrose, glucose, dextrose, lactose, and maltodextrin; natural and synthetic gums; gelatin; cellulose derivatives such as microcrystalline cellulose, hydroxypropyl cellulose, hydroxyethyl cellulose, hydroxypropyl methyl cellulose, carboxymethyl cellulose, methyl cellulose, and ethyl cellulose; polyvinylpyrrolidone (polyvinylpyrrolidone); polyethylene glycol (PEG); waxes; calcium carbonate; calcium phosphate; alcohols such as sorbitol, xylitol, mannitol, and water, or combinations thereof.

In some embodiments, suitable lubricants include metal stearate salts (e.g., magnesium stearate, calcium stearate, aluminum stearate), fatty acid esters (e.g., sodium stearoyl fumarate), fatty acids (e.g., stearic acid), fatty alcohols, glyceryl betaine, mineral oil, paraffin wax, hydrogenated vegetable oil, leucine, polyethylene glycol (PEG), metal dodecyl sulfate salts (e.g., sodium dodecyl sulfate, magnesium dodecyl sulfate), sodium chloride, sodium benzoate, sodium acetate, and talc, or combinations thereof.

In some embodiments, the disintegrant may be a non-effervescent disintegrant. Suitable non-effervescent disintegrants include starches such as corn starch, potato starch, pregelatinized and modified starches, sweeteners, clays such as bentonite, microcrystalline cellulose, alginate, sodium glycolate, gums such as agar, guar gum, locust bean gum, karaya gum, pectin, and tragacanth gum. In some embodiments, the disintegrant may be an effervescent disintegrant. Suitable effervescent disintegrants include sodium bicarbonate in combination with citric acid and sodium bicarbonate in combination with tartaric acid.

In some embodiments, suitable flavoring agents may be selected from cinnamon oil; wintergreen oil; peppermint oil; clover oil; hay oil; anise oil; eucalyptus oil; vanilla; citrus oils such as lemon oil, orange oil, grape and grapefruit oil; and fruit essences including apple, peach, pear, strawberry, raspberry, cherry, plum, pineapple, and apricot essences.

In some embodiments, suitable sweeteners include glucose (corn syrup), dextrose, invert sugar, fructose and mixtures thereof (when not used as a carrier); saccharin and its various salts, such as sodium salts; dipeptide sweeteners such as aspartame; dihydrochalcone compounds, glycyrrhizin; stevia (stevia rebaudiana) (stevioside); chlorinated derivatives of sucrose, such as sucralose; and sugar alcohols such as sorbitol, mannitol, xylitol, etc.

In some embodiments, suitable colorants include food, pharmaceutical, and cosmetic pigments (FD&C), pharmaceutical and cosmetic pigments (D&C), and external pharmaceutical and cosmetic pigments (Ext.D&C).

In some embodiments, suitable chelating agents include ethylenediamine-*N,N,N',N'*-tetraacetic acid (EDTA); the disodium, trisodium, tetrasodium, dipotassium, tripotassium, dilithium, and diammonium salts of EDTA; the barium, calcium, cobalt, copper, dysprosium, europium, iron, indium, lanthanum, magnesium, manganese, nickel, samarium, strontium, or zinc chelates of EDTA; trans-1,2-diaminocyclohexane-*N,N,N',N'*-tetraacetic acid monohydrate; *N,N*-bis(2-hydroxyethyl)glycine; 1,3-diamino-2-hydroxypropane-*N,N,N',N'*-tetraacetic acid; 1,3-diaminopropane-*N,N,N',N'*-tetraacetic acid; ethylenediamine-*N,N'*-diacetic acid; ethylenediamine-*N,N'*-dipropionic acid dihydrochloride; ethylenediamine-*N,N'*-bis(methylenephosphonic acid) hemihydrate; *N*-(2-hydroxyethyl)ethylenediamine-*N,N',N'*-triacetic acid; ethylenediamine-*N,N,N',N'*-tetrakis(methylenephosphonic acid); *O,O'*-bis(2-aminoethyl)ethylene glycol-*N,N,N',N'*-tetraacetic acid; *N,N*-bis(2-hydroxybenzyl)ethylenediamine-*N,N-*diacetic acid; 1,6-hexamethylenediamine-*N,N,N',N'*-tetraacetic acid; *N*-(2-hydroxyethyl)iminodiacetic acid; iminodiacetic acid; 1,2-diaminopropane-*N,N,N',N'*-tetraacetic acid; nitrilotriacetic acid; nitrilotripropionic acid; the trisodium salt of nitrilotris(methylenephosphonic acid); 7,19,30-trioxa-1,4,10,13,16,22,27,33-octaazabicyclo[11,11,11]pentatriacontane hexahydrobromide; or triethylenetetramine-*N,N,N',N",N‴,N‴*-hexaacetic acid, and the like.

In some embodiments, suitable diluents include water, glycerol, methanol, ethanol, and other biocompatible diluents.

In some embodiments, suitable surfactants include polysorbate, sodium lauryl sulfate, sodium stearoyl fumarate, polyoxyethylene alkyl ethers, sorbitan fatty acid esters, polyethylene glycol (PEG), polyoxyethylene castor oil derivatives, glycol esters of fatty acids, glycerides of fatty acids, or combinations thereof.

In some embodiments, the pharmaceutical composition may further comprise a second active pharmaceutical ingredient. In some embodiments, the second active pharmaceutical ingredient is an epidermal growth factor receptor (EGFR) inhibitor. In some embodiments, the EGFR inhibitor is selected from paclitaxel, cisplatin, cytarabine (AraC), erlotinib, and nimotuzumab.

The polypeptide of the present application may be formulated into various dosage forms and administered via various routes, such as oral, rectal, or parenteral administration. The term "parenteral" as used herein may include intra-arterial, intracardiac, intraventricular, intradermal, intraduodenal, intramedullary, intramuscular, intraosseous, intraperitoneal, intrathecal, intravenous, intravitreal, epidural, subcutaneous, inhalation, transdermal, transmucosal, sublingual, buccal, and topical (including epidermal, dermal, enema, eye drops, ear drops, intranasal, and vaginal) administration. In some exemplary embodiments, the route of administration may be by injection, such as intramuscular, intravenous, subcutaneous, or intraperitoneal injection. Formulations for oral administration may include capsules, tablets, caplets, pills, lozenges, troches, powders, and granules, among others.

In another aspect, the present application provides a kit, which comprises the non-natural polypeptide or the pharmaceutical composition as described in any of the preceding claims, and optionally a second therapeutic agent.

In some embodiments, the second therapeutic agent is an epidermal growth factor receptor (EGFR) inhibitor. In some embodiments, the EGFR inhibitor is selected from the group consisting of paclitaxel, cisplatin, cytarabine (AraC), erlotinib, and nimotuzumab. In some embodiments, the non-natural polypeptide or the pharmaceutical composition and the second therapeutic agent are placed in the same or different formulation units.

In some embodiments, the kit is used for:
(1) inhibiting proliferation or metastasis of a tumor cell, or promoting apoptosis of the tumor cell; preferably, the tumor cell is selected from the group consisting of prostate cancer cells, ovarian cancer cells, pancreatic cancer cells, colon cancer cells, small cell lung cancer (SCLC) cells, non-small cell lung cancer (NSCLC) cells, breast cancer cells, renal cancer cells, melanoma cells, multiple myeloma cells, glioblastoma cells, Burkitt lymphoma cells, leukemia cells, squamous cell carcinoma (e.g., laryngeal squamous cell carcinoma) cells, adenocarcinoma (e.g., vulvar adenocarcinoma) cells, liver cancer, and cervical cancer cells;
(2) inhibiting angiogenesis, tumor growth or metastasis;
(3) treating a disease associated with CK2 activity (e.g., a tumor).

In some embodiments, the tumor is a refractory cancer or a cancer insensitive to the CIGB-300 peptide. Preferably, the tumor is selected from the group consisting of prostate cancer, ovarian cancer, pancreatic cancer, colon cancer, small cell lung cancer (SCLC), non-small cell lung cancer (NSCLC), breast cancer, renal cancer, melanoma, multiple myeloma, glioblastoma, Burkitt lymphoma, leukemia, squamous cell carcinoma (e.g., laryngeal squamous cell carcinoma), adenocarcinoma (e.g., vulvar adenocarcinoma), liver cancer, and cervical cancer.

### Use

The effect of the polypeptide described herein in promoting tumor cell death was tested using cell lines from different sources. Apoptosis was measured using methods such as sulforhodamine, alamar blue, or crystal violet as reported in the literature, and the results were expressed as half-maximal inhibitory concentration (IC₅₀) (Boyd et al., 1995). The IC₅₀ value refers to the peptide dose that induces 50% apoptosis, obtained from a dose-response curve. The effect of the polypeptide at doses ranging from 0 to 200 µM was tested on various tumor cell lines. In some embodiments, the lipopeptide exhibited at least a 4-fold increase in potency against tumor cells compared to the CIGB-300 peptide, indicating that the lipopeptide achieved the same tumor cell-killing effect with only one-fourth of the dose of the CIGB-300 peptide, significantly reducing drug dosage and improving safety.

Using annexin-V, a biomarker of apoptosis, as an indicator of cell death, short-term time-course comparative experiments were conducted. Flow cytometry analysis revealed that the lipopeptide also exhibited significant advantages. In some embodiments, based on the percentage of apoptotic cells, the lipopeptide induced higher levels of early apoptosis compared to the CIGB-300 peptide. In other embodiments, treatment with the lipopeptide resulted in elevated reactive oxygen species (ROS) levels. Additionally, non-tumorigenic normal-like cells from mice demonstrated greater resistance to the lipopeptide.

The antitumor effects of the polypeptide were validated through head-to-head experimental in vivo metastasis assays. In some embodiments, C57BL6 mice were intravenously injected with mouse Lewis lung carcinoma 3LL cells. Seventy-two hours after tumor cell challenge, the lipopeptide and the CIGB-300 peptide were administered intravenously for five consecutive days. Thirty days later, pulmonary metastatic foci were quantified. The results showed that even at one-tenth of the dose of the CIGB-300 peptide, the lipopeptide still exhibited effective anti-metastatic activity, and no systemic toxicity was observed.

In addition, the targeting effect of the polypeptide on CK2 signal transduction was examined. In some embodiments, after incubating IC₅₀ doses of the lipopeptide and the CIGB-300 peptide with tumor cell lines, endogenous CK2 enzyme activity in cell extracts was detected. The results showed that the lipopeptide inhibited CK2 enzyme activity by more than 50% in all tumor cells. Western blot (WB) experiments revealed that, compared to the CIGB-300 peptide, the lipopeptide exhibited stronger inhibitory effects on phosphorylation and CK2-related proteins such as cdc37. These findings indicate that the lipopeptide interacts more strongly with the RPS6 protein than the CIGB-300 peptide, enabling it to more effectively inhibit CK2-mediated phosphorylation and induce a higher internalization rate in tumor cells.

Based on the above-mentioned data, the peptide and the pharmaceutical composition as described herein may be administered to a subject to at least partially ameliorate a disease. In some embodiments, the subject may have been previously diagnosed with the disease described herein or may be at risk of developing the disease described herein.

In one aspect, the present application provides a use of the non-natural polypeptide, the pharmaceutical composition, or the kit as described in any preceding items in the manufacture of a CK2 modulator (e.g., an inhibitor) or in the manufacture of a medicament, wherein the medicament is used for:
(1) inhibiting proliferation or metastasis of a tumor cell, or promoting apoptosis of the tumor cell;
(2) inhibiting angiogenesis, tumor growth or metastasis;
(3) treating a disease associated with CK2 activity (e.g., a tumor).

In another aspect, the present application provides a method for modulating (e.g., inhibiting) CK2 activity in a cell, the method comprising contacting the cell with the non-natural polypeptide, the pharmaceutical composition, or the kit as described in any one of the preceding items.

In another aspect, the present application provides a method for inhibiting proliferation or metastasis of a tumor cell, or promoting apoptosis of the tumor cell, wherein the method comprises administering to the subject an effective amount of the non-natural polypeptide, the pharmaceutical composition, or the kit as described in any one of the preceding items.

In another aspect, the present application provides a method for inhibiting angiogenesis, or inhibiting tumor growth or metastasis, wherein the method comprises administering to the subject an effective amount of the non-natural polypeptide, the pharmaceutical composition, or the kit as described in any one of the preceding items.

In another aspect, the present application provides a method for treating a disease associated with CK2 activity in a subject, wherein the method comprises administering to the subject a therapeutically effective amount of the non-natural polypeptide, the pharmaceutical composition, or the kit as described in any one of the preceding items.

In any of the foregoing aspects, the cell is a tumor cell. In some embodiments, the tumor cell is a cell line or a cell derived from the subject. In some embodiments, the tumor cell is selected from the group consisting of prostate cancer cells, ovarian cancer cells, pancreatic cancer cells, colon cancer cells, small cell lung cancer (SCLC) cells, non-small cell lung cancer (NSCLC) cells, breast cancer cells, renal cancer cells, melanoma cells, multiple myeloma cells, glioblastoma cells, Burkitt lymphoma cells, leukemia cells, squamous cell carcinoma (e.g., laryngeal squamous cell carcinoma) cells, adenocarcinoma (e.g., vulvar adenocarcinoma) cells, liver cancer cells and cervical cancer cells.

In any of the foregoing aspects, the disease associated with CK2 activity is a tumor. In some embodiments, the tumor is a refractory cancer or a cancer insensitive to the CIGB-300 peptide. In some embodiments, the tumor is selected from the group consisting of prostate cancer, ovarian cancer, pancreatic cancer, colon cancer, small cell lung cancer (SCLC), non-small cell lung cancer (NSCLC), breast cancer, renal cancer, melanoma, multiple myeloma, glioblastoma, Burkitt lymphoma, leukemia, squamous cell carcinoma (e.g., laryngeal squamous cell carcinoma), adenocarcinoma (e.g., vulvar adenocarcinoma), liver cancer, and cervical cancer.

In any of the aforementioned methods, further comprising administering to the subject a therapeutically effective amount of a second therapeutic agent in combination therewith. In some embodiments, the second therapeutic agent is an epidermal growth factor receptor (EGFR) inhibitor. In some embodiments, the EGFR inhibitor is selected from the group consisting of paclitaxel, cisplatin, cytarabine (AraC), erlotinib, and nimotuzumab. In some embodiments, the non-natural polypeptide or the pharmaceutical composition is administered simultaneously or sequentially with the second therapeutic agent.

### Beneficial technical effects

The lipopeptide described herein, comprising decanoic acid chemically conjugated to the N-terminus of the CIGB-300 peptide, is not only a novel anti-cancer compound but also exhibits more potent anti-cancer activity than the CIGB-300 peptide. In vitro experiments on tumor cell lines have confirmed the advantages of the lipopeptide, demonstrating that the dose required to kill 50% of the cells was at least four times lower than that required for the CIGB-300 peptide. At a dose one-tenth that of the CIGB-300 peptide, the lipopeptide achieved effective anti-metastatic effects. At the molecular level, the lipopeptide's advantage over the CIGB-300 peptide resides in its more effective inhibition of CK2 enzyme activity. Additionally, the lipopeptide exhibited stronger targeting specificity toward the RPS6 protein.

The lipopeptide described herein is obtained by a simple chemical modification involving the conjugation of decanoic acid to the terminus of the CIGB-300 peptide, thereby overcoming the baseline or intrinsic resistance of tumor cells to the CIGB-300 peptide. Consequently, the lipopeptide described herein is a novel and more effective anti-cancer compound that allows reduced pharmacological doses without increased toxicity.

Compared to the CIGB-300 peptide, the lipopeptide described herein achieves a more potent antitumor effect at lower doses, providing significant reference value for the clinical use of anti-cancer drugs in cancer patients. Given that the synthetic method disclosed in the present application is consistent with the synthesis method used to obtain clinical batches of the CIGB-300 peptide, large-scale production is feasible.

### Specific Models for Carrying Out the present invention

The embodiments of the present invention will be described in detail below with reference to the examples. However, those skilled in the art will understand that the following examples are for illustrative purposes only and should not be considered as limiting the scope of the present invention. Unless otherwise specified, specific conditions in the examples were performed under conventional conditions or conditions recommended by the manufacturer. Reagents or instruments used without specified manufacturers were all commercially available conventional products.

### Summary table of sequence information

| SEQ ID NO: | Description | Sequence |
|---|---|---|
| 1 | Lipopeptide | X₁-GRKKRRQRRRPPQXCWMSPRHLGTC-NH₂ |
| | | X₁ represents decanoyl (-CO(CH₂)₈CH₃), X represents β-Ala (3-aminopropionic acid), and a disulfide bond is formed between the cysteines at position 15 and position 25. |
| 2 | X₂ domain | GRKKRRQRRRPPQ |
| 3 | X₃ domain | CWMSPRHLGTC |

### Example 1: Preparation method of the peptide

The CIGB-300 peptide was synthesized via the solid-phase method based on Fmoc/tBu chemistry, purified by reversed-phase high-performance liquid chromatography using an acetonitrile/H₂O-trifluoroacetic acid gradient to a purity exceeding 95%, and subsequently characterized by ion spray mass spectrometry.

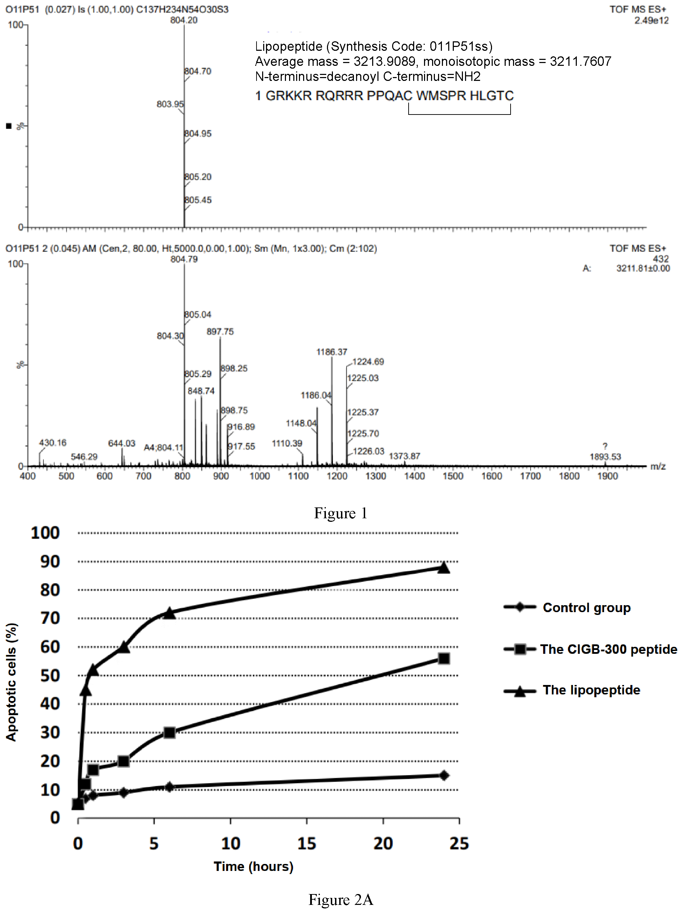

The lipopeptide was obtained by conjugating a lipophilic carboxylic acid (decanoic acid, C₁₀H₂₀O₂) at the N-terminus of the CIGB-300 peptide. The MS detection results are shown in Figure 1: average mass = 3213.9089, monoisotopic mass = 3211.7607.

### Example 2: Direct comparative experiment on antitumor cell effects of lipopeptide and the CIGB-300 peptide

All human tumor cells used in the present application were obtained from the American Type Culture Collection (ATCC, Manassas, VA, USA) and cultured in RPMI 1640 medium (Gibco) supplemented with 10% (v/v) fetal bovine serum and gentamicin (50 µg/ml) at 37 °C in a 5% CO₂ atmosphere. The effects of the lipopeptide and the CIGB-300 peptide on tumor cell viability were assessed using Alamar Blue reagent (Life Technologies, Carlsbad, CA, USA). Cells were seeded in flatbottom 96-well plates (2×10⁵ cells/mL, 200 µL/well) and incubated for 24 hours, followed by treatment with the lipopeptide and the CIGB-300 peptide serially diluted from 100 to 3.12 µM at a 1:2 ratio. After 48 hours of incubation, 10% (v/v) Alamar Blue was added, and the cell suspension was incubated for an additional 4 hours. Fluorescence intensity in the microplates was measured, and IC₅₀ values were calculated using CalcuSyn software (version 2.1) (Biosoft, Cambridge, UK).

The data in Table 1 indicate that the lipopeptide was at least 4 times more potent than the CIGB-300 peptide against all types of tumor cells. In contrast, benign tumor cells appeared to be more resistant to the cell death induced by the lipopeptide. In the same benign tumor cells, the cytotoxicity induced by the lipopeptide was no more than 1.5-fold that induced by the CIGB-300 peptide.

**Table 1: Effects of the lipopeptide and the CIGB-300 peptide on viability of tumor cells in vitro**

| Cell line | Phenotype | Tissue origin | IC₅₀ value (µM) | | Ratio |
|---|---|---|---|---|---|
| | | | The CIGB-300 peptide | The lipopeptide | |
| NCI-H82 | Malignant | SCLC | 39.7±11 | 4.4±0.2 | 9.02 |
| NCI-H125 | Malignant | NSCLC | 76.3±2 | 19±0.7 | 4.02 |
| NCI-H460 | Malignant | NSCLC | 30.0±9 | 5.0±4 | 6.00 |
| NCI-H226 | Malignant | NSCLC | 70.8 | 18±6 | 3.93 |
| NCI-H522 | Malignant | NSCLC | 70.0 | 11±3 | 6.36 |
| SK-MES1 | Malignant | NSCLC | 150±7 | 28±4 | 5.36 |
| A549 | Malignant | NSCLC | 180±30 | 30±10 | 6.00 |
| SW948 | Malignant | Colon adenocarcinoma | 143.1±1 | 11.6±1.2 | 12.34 |
| PC3 | Malignant | Prostate cancer | 169.9±5 | 38±1.5 | 4.47 |
| U87 | Malignant | Glioblastoma | 154.3±15 | 30±7 | 5.14 |
| A431 | Malignant | Vulvar adenocarcinoma | 90.7±4 | 18.1±3 | 5.01 |
| HepG2 | Malignant | Hepatocellular carcinoma | 127.7±44 | 15.7±2 | 8.13 |
| Hep-2 | Malignant | Laryngeal squamous cell carcinoma | 30.7±2 | 6.0±1 | 5.12 |
| MRC-5 | Benign | Lung epithelial cells | 198±8 | 169±12 | 1.17 |
| RR-805 | Benign | Renal primary fibroblasts | 230±6 | 204±10 | 1.13 |

| | | | | | |
|---|---|---|---|---|---|
| Note: SCLC refers to small cell lung cancer; NSCLC refers to non-small cell lung cancer. | | | | | |

### Example 3: Time-course experiment on apoptosis and reactive oxygen species induced by the lipopeptide and the CIGB-300 peptide

Apoptosis induced by the lipopeptide and the CIGB-300 peptide in five tumor cell lines was detected using the Annexin V-FITC Apoptosis Detection Kit I (BD Biosciences, San Jose, CA, USA).

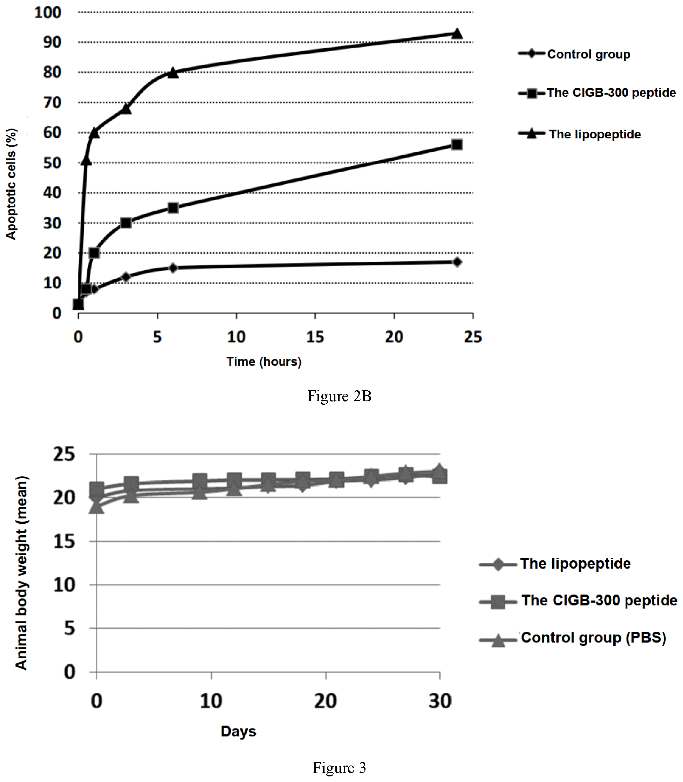

The specific experimental procedure involved incubating the cells with equivalent doses of the lipopeptide and the CIGB-300 peptide for 0.5 h, 1 h, 3 h, 6 h, and 24 h. After incubation, the cells were washed twice with ice-cold PBS and resuspended in 1× binding buffer at a final concentration of 1×10⁵ cells/mL. Subsequently, 5 µL of Annexin V-FITC apoptosis detection reagent and propidium iodide (PI) were added, and the cell suspension was incubated at room temperature in the dark for 15 minutes. Stained cells were analyzed by flow cytometry using a Partec CyFlow Space instrument, with data analysis and visualization performed using FlowJo software (version 7.6.1) (BD, Ashland, OR, USA). Figures 2A and 2B show the percentages of apoptotic cells induced in NCI-H226 cells and SK-MES1 cells, respectively, demonstrating that the lipopeptide induced higher apoptosis rates compared to the CIGB-300 peptide.

In addition to inducing higher levels of apoptosis compared to the CIGB-300 peptide, the lipopeptide also triggered greater reactive oxygen species (ROS) production in A549 cells after 2 hours of incubation with the antioxidant *N*-acetyl-*L*-cysteine (NAC), as analyzed by flow cytometry (Table 2). The elevated ROS levels were correlated with the stronger inhibitory effect of the lipopeptide on cell viability.

**Table 2: ROS levels induced by the lipopeptide and the CIGB-300 peptide in A549 cells**

| Treatment group | | | Control group | The CIGB-300 peptide | The lipopeptide | H₂O₂ |
|---|---|---|---|---|---|---|
| Without NAC | Cell viability (%) | Mean | 93.9 | 78.9 | 72.8 | 94.0 |
| | | Standard deviation | 0.1 | 0.1 | 1.7 | 0.3 |
| | DHE reagent (Geometric mean) | Mean | 3978 | 4446 | 4361 | 4335 |
| | | Standard deviation | 101 | 19 | 17 | 12 |
| With NAC | Cell viability (%) | Mean | 94.1 | 85.4 | 60.6 | 94.9 |
| | | Standard deviation | 0.4 | 3.0 | 2.0 | 0.6 |
| | DHE reagent (Geometric mean) | Mean | 4313 | 4355 | 3501 | 4175 |
| | | Standard deviation | 79 | 36 | 12 | 259 |

### Example 4: Head-to-head comparative experiment evaluating anti-metastatic effects of the lipopeptide and the CIGB-300 peptide

The specific experimental procedure involved inducing experimental metastasis via tail vein injection of 2×10⁵ Lewis mouse lung carcinoma 3LL cells for a lung colonization assay. The lipopeptide or the CIGB-300 peptide was administered intravenously at doses of 0.1, 1, 5, and 10 mg/kg for 5 consecutive days, with 10 mice per group. The control group received an intravenous injection of PBS. On Day 30, the animals were euthanized, and the lungs were excised and fixed in Bouin's solution. Metastatic pulmonary nodules were counted using a dissecting microscope. Additionally, animal body weight and signs of toxicity were monitored throughout the experiment.

The data in Table 3 indicate that, compared with the CIGB-300 peptide, the lipopeptide significantly reduced lung colonization at all dose levels. In contrast, the CIGB-300 peptide exerted antimetastatic effects only at the highest dose (10 mg/kg).

This head-to-head comparison demonstrates that the lipopeptide produced significant antimetastatic effects at one-twentieth of the dose of the CIGB-300 peptide. At 10 mg/kg, the lipopeptide achieved 100% inhibition of lung metastasis. No signs of toxicity were observed in the treatment group receiving the highest dose (10 mg/kg), and animal body weight increased during the experimental period (Figure 3).

**Table 3: Antimetastatic effects of the lipopeptide and the CIGB-300 peptide in direct comparative animal experiment**

| Treatment group | Dose level (mg/kg) | Lung metastasis amount (mean standard deviation) |
|---|---|---|
| The lipopeptide | 0.1 | 30±9*** |
| | 1.0 | 12±6*** |
| | 5.0 | 4±4*** |
| | 10.0 | 0 |
| The CIGB-300 peptide | 0.1 | 171±37 |
| | 1.0 | 155±29 |
| | 5.0 | 130±35 |
| | 10.0 | 96±19* |
| Control group (PBS) | | 160±42 |

| | | |
|---|---|---|
| Note: Bilateral pulmonary nodules were counted for each animal, and the mean of 10 animals per group is shown. ***(*p<*0.05), **(*p*<0.01), ***(*p*<0.001), Mann-Whitney U test. | | |

### Example 5: Effects of the lipopeptide and the CIGB-300 peptide on CK2 enzyme activity and CK2 signal transduction

A CK2 radioactive assay was performed with increasing concentrations of the lipopeptide solution (the final volume was 18 µL), using the CIGB-300 peptide solution or an equivalent amount of dimethyl sulfoxide (DMSO) as controls. 3 µL of recombinant CK2α (28 ng) was mixed with a solution containing 1 mM specific CK2 peptide substrate "RRREEETEEE" (Promega), 10 mM MgCl₂, 1 µCi ³²P-γATP (6000 Ci/mmol), and 100 µM ATP. After incubation at room temperature for 5 minutes, the reaction was terminated by adding 60 µL of 4% trichloroacetic acid. Subsequently, 5 µL of the resulting reaction solution was spotted onto Whatman PE-81 filter paper, which was then washed four times with 10 mM H₃PO₄. Finally, the radioactivity retained on the filter paper was quantified, with counts per minute (CPM) representing CK2 enzyme activity.

The data in Table 4 show that the lipopeptide inhibited CK2 enzyme activity more effectively than the CIGB-300 peptide, indicating that the lipopeptide of the present invention exhibited more potent target interaction.

**Table 4: Head-to-head comparative assay for CK2 enzyme activity inhibition**

| Peptide concentration (µM) | CK2 enzyme activity (CPM x10³) | |
|---|---|---|
| | CIGB-300 peptide | Lipopeptide |
| 50 | 4 ±4 | 0.19 |
| 25 | 19 ±8 | 0.20 |
| 12.5 | 31 ±7 | 0.46 |
| 6.25 | 61 ±10 | 1 |
| 3.125 | 92 ±12 | 3 |
| 1.56 | 109 ±9 | 16 ±9 |
| 0.781 | 158 ±14 | 39 ±12 |
| 0.39 | 162 ±12 | 62 ±14 |
| 0.195 | 169 ±21 | 101 ±10 |
| 0.097 | 171±18 | 169 ±12 |
| 0.00 | 170 ±24 | 174 ±20 |

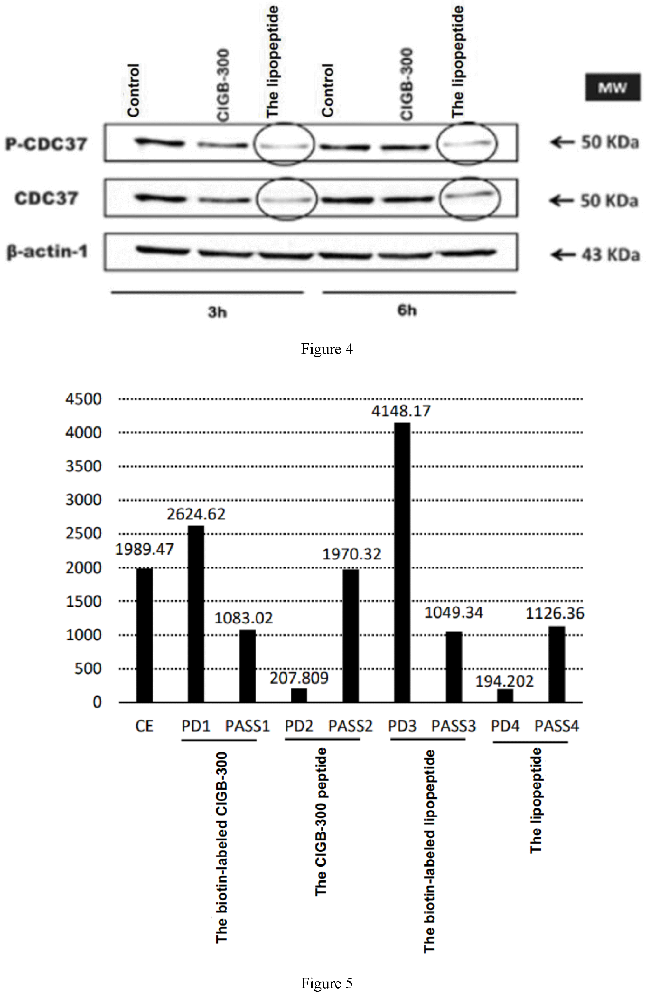

Based on the CK2 enzyme activity data, a head-to-head comparative experiment was performed for the lipopeptide and the CIGB-300 peptide using the lung squamous cell carcinoma cell line NCI-H226. The experiment assessed the lipopeptide's ability to inhibit other CK2 signaling substrates, such as cdc37 protein. Analysis of the western blotting results shown in Figure 4 confirmed that, compared with the CIGB-300 peptide, the lipopeptide more effectively inhibited phosphorylation and reduced cdc37 protein levels following 3 and 6 hours of incubation in the NCI-H226 cell line.

To investigate the targeting effects of the lipopeptide and the CIGB-300 peptide on CK2 signaling, an RPS6 protein pull-down assay was performed. The specific experimental setting included seeding 3,600,000 NCI-H226 cells into a T-75 flask. After 24 hours, the cells were harvested and washed, then lysed using 1× PBS containing 1 mM DTT (Sigma, St. Louis, MO, USA), Triton X-100 (1%), and cOmplete^{™} protease inhibitor (Roche, Basel, Switzerland). Cell lysates were clarified by centrifugation, and 300 µg of total protein were incubated with 100 µM of biotin-labeled lipopeptide, biotin-labeled CIGB-300 peptide, or culture medium at room temperature for 30 minutes, followed by an addition to 30 µL of pre-equilibrated streptavidin-agarose matrix (GE Healthcare, Chicago, IL, USA). After incubation at 4 °C for 1 hour, the matrix was collected by centrifugation and washed thoroughly with ice-cold PBS containing 1 mM DTT. The pulled-down RPS6 protein was eluted, separated by SDS-PAGE, analyzed by western blotting, and the specific signals were quantified by densitometry. The data presented in Figure 5 demonstrate that the lipopeptide exhibited a superior targeting effect on RPS6 protein (PD3) as compared to the CIGB-300 peptide (PD1).

Using the lung squamous cell carcinoma cell line NCI-H226, a head-to-head comparative experiment was conducted between the lipopeptide and the CIGB-300 peptide with respect to their targeted proteins. Pull-down assays and mass spectrometry analysis were performed. The data in Table 5 reveal the proteins targeted by the lipopeptide, while the data in Table 6 show the proteins more effectively targeted by the lipopeptide as compared to the CIGB-300 peptide.

**Table 5: Proteins targeted by the lipopeptide in NCI-H226 cell line**

| **Accession code** | **Description** |
|---|---|
| **Q9ULD0** | 2-oxoglutarate dehydrogenase-like, mitochondrial OS=Homo sapiens OX=9606 GN=OGDHL PE=1 SV=3 |
| **Q66LE6** | Serine/threonine-protein phosphatase 2A 55 kDa regulatory subunit B delta isoform OS=Homo sapiens OX=9606 GN=PPP2R2D PE=1 SV=1 |
| **015514** | DNA-directed RNA polymerase I subunit RPB4 OS=Homo sapiens OX=9606 GN=POLR2D PE=1 SV=1 |
| **Q6NUQ4** | Transmembrane protein 214 OS=Homo sapiens OX=9606 GN=TMEM214 PE=1 SV=2 |
| **Q71D13** | Histone H3.2 OS=Homo sapiens OX=9606 GN=H3C13 PE=1 SV=3 |
| **P49643** | DNA primase large subunit OS=Homo sapiens OX=9606 GN=PRIM2 PE=1 SV=2 |
| **P68431** | Histone H3.1 OS=Homo sapiens OX=9606 GN=H3C12 PE=1 SV=2 |
| **Q5QNW6** | Histone H2B type 2-F OS=Homo sapiens OX=9606 GN=H2BC18 PE=1 SV=3 |

**Table 6: Proteins more effectively targeted by the lipopeptide than the CIGB-300 peptide in NCI-H226 cell line**

| **Accession code** | **Description** | **Abundance ratio of lipopeptide/CIGB-300** |
|---|---|---|
| **Q12996** | Cleavage stimulation subunit 3 OS=Homo sapiens OX=9606 GN=CSTF3 SV=1 | 5.12 |
| **P52926** | High mobility group protein HMGI-C OS=Homo sapiens OX=9606 GN=HMGA2 PE=1 SV=1 | 3.877 |
| **O95777** | U6 snRNA-associated Sm-like protein LSm8 OS=Homo sapiens OX=9606 GN=LSM8 PE=1 SV=3 | 3.308 |
| **P20908** | Collagen alpha-1(V) chain OS=Homo sapiens OX=9606 GN=COL5A1 PE=1 SV=1 | 2.971 |
| **Q9BRL6** | Serine/arginine-rich splicing factor 8 OS=Homo sapiens OX=9606 GN=SRSF8 PE=1 SV=1 | 2.857 |
| **O14893** | Gem-associated protein 2 OS=Homo sapiens OX=9606 GN=GEMIN2 PE=1 SV=1 | 2.616 |
| **Q15417** | Calponin-3 OS=Homo sapiens OX=9606 GN=CUL1 | 2.367 |
| **Q13616** | Cullin-1 OS=Homo sapinopeptidase 1 OS=Homo sapiens OX=9606 GN=XPNPEP1 PE=1 SV=3 | 2.302 |
| **Q9NAW7** | Xaa-Pro aminopeptidase 1 OS=Homo sapiens OX=9606 GN=XPNPEP1 PE=1 SV=3 | 2.262 |
| **Q8IUE6** | Histone H2A type 2-B OS=Homo sapiens OX=9606 GN=XPNPEP1 PE=1 SV=3 | 2.192 |
| **P15927** | Replication protein A 32 kDa subunit OS=Homo sapiens OX=9606 GN=RPA2 PE=1 SV=1 | 2.103 |
| **Q15527** | Surfeit locus protein 2 OS=Homo sapiens OX=9606 GN=SURF2 PE=1 SV=4 | 2.052 |
| **O95260** | Arginyl-tRNA-protein transferase 1 OS=Homo sapiens OX=9606 GN=ATE1 PE=1 SV=2 | 2.013 |

Although specific embodiments of the present invention have been described in detail, those skilled in the art will appreciate that various modifications and substitutions can be made to these details based on all the teachings disclosed herein, and all such changes fall within the scope of protection of the present invention. The full scope of the present invention is defined by the appended claims and any equivalents thereof.

## Claims

1. A non-natural polypeptide, comprising domains X₁, X₂, and X₃, wherein:
X₁ is selected from C₆-C₂₀ fatty acid residues;
X₂ is selected from fragments of a cell-penetrating peptide (CPP);
X₃ is selected from fragments of a peptide capable of specifically binding to protein kinase 2 (CK2).

2. The non-natural polypeptide according to claim 1, wherein X₁ is selected from C₆-C₁₂ fatty acid residues (e.g., C₆, C₇, C₈, C₉, C₁₀, C₁₁, or C₁₂ fatty acid residues);
preferably, X₁ is decanoyl.

3. The non-natural polypeptide according to claim 1 or 2, wherein X₂ comprises a fragment of a peptide having an identity of at least 50%, 55%, 60%, 65%, 70%, 75%, 80%, 85%, 90%, 95%, 98%, or 100% as compared with GRKKRRQRRRPPQ (SEQ ID NO: 2).

4. The non-natural polypeptide according to any one of claims 1 to 3, wherein X₃ has CK2 inhibitory activity, such as inhibiting CK2 phosphorylation;
preferably, X₃ comprises a fragment of a peptide having an identity of at least 50%, 55%, 60%, 65%, 70%, 75%, 80%, 85%, 90%, 95%, 98%, or 100% as compared with CWMSPRHLGTC (SEQ ID NO: 3, wherein a disulfide bond is formed between the cysteines at position 1 and position 11).

5. The non-natural polypeptide according to any one of claims 1 to 4, wherein an amino acid or a peptide linker exists between two adjacent domains of X₁, X₂, and X₃.

6. The non-natural polypeptide according to any one of claims 1 to 5, wherein X₁, X₂, and X₃ are linked in any order;
preferably, X₁, X₂, and X₃ are arranged sequentially from the N-terminus to the C-terminus; preferably, the polypeptide is connected from the N-terminus to the C-terminus in the order of X₁-X₂-β-Ala-X₃.

7. The non-natural polypeptide according to any one of claims 1 to 6, which comprises or is composed of the following amino acid sequence:
X₁-Gly¹-Arg²-Lys³-Lys⁴-Arg⁵-Arg⁶-Gln⁷-Arg⁸-Arg⁹-Arg¹⁰-Pro¹¹-Pro¹²-Gln¹³-β-Ala¹⁴-Cys¹⁵-Trp¹⁶-Met¹⁷-Ser¹⁸-Pro¹⁹-Arg²⁰-His²¹-Leu²²-Gly²³-Thr²⁴-Cys²⁵-NH₂ (SEQ ID NO: 1), wherein,
X₁ is decanoyl;
a disulfide bond is formed between Cys¹⁵ and Cys²⁵.

8. A pharmaceutical composition, which comprises the non-natural polypeptide according to any one of claims 1 to 7 and a pharmaceutically acceptable excipient.

9. A kit, which comprises the non-natural polypeptide according to any one of claims 1 to 7 or the pharmaceutical composition according to claim 8, and optionally a second therapeutic agent;
preferably, the second therapeutic agent is an epidermal growth factor receptor (EGFR) inhibitor, more preferably, the EGFR inhibitor is selected from the group consisting of paclitaxel, cisplatin, cytarabine (AraC), erlotinib, and nimotuzumab;
preferably, the non-natural polypeptide or the pharmaceutical composition and the second therapeutic agent are placed in the same or different formulation units;
preferably, the kit is used for:
(1) inhibiting proliferation or metastasis of a tumor cell, or promoting apoptosis of the tumor cell; preferably, the tumor cell is selected from the group consisting of prostate cancer cells, ovarian cancer cells, pancreatic cancer cells, colon cancer cells, small cell lung cancer (SCLC) cells, non-small cell lung cancer (NSCLC) cells, breast cancer cells, renal cancer cells, melanoma cells, multiple myeloma cells, glioblastoma cells, Burkitt lymphoma cells, leukemia cells, squamous cell carcinoma (e.g., laryngeal squamous cell carcinoma) cells, adenocarcinoma (e.g., vulvar adenocarcinoma) cells, liver cancer cells, and cervical cancer cells;
(2) inhibiting angiogenesis, tumor growth or metastasis;
(3) treating a disease associated with CK2 activity (e.g., a tumor);
preferably, the tumor is a refractory cancer or a cancer insensitive to the CIGB-300 peptide, and preferably, the tumor is selected from the group consisting of prostate cancer, ovarian cancer, pancreatic cancer, colon cancer, small cell lung cancer (SCLC), non-small cell lung cancer (NSCLC), breast cancer, renal cancer, melanoma, multiple myeloma, glioblastoma, Burkitt lymphoma, leukemia, squamous cell carcinoma (e.g., laryngeal squamous cell carcinoma), adenocarcinoma (e.g., vulvar adenocarcinoma), liver cancer, and cervical cancer.

10. Use of the non-natural polypeptide according to any one of claims 1 to 7, the pharmaceutical composition according to claim 8, or the kit according to claim 9, in the manufacture of a CK2 modulator (e.g., an inhibitor), or in the manufacture of a medicament, wherein the medicament is used for:
(1) inhibiting proliferation or metastasis of a tumor cell, or promoting apoptosis of the tumor cell;
(2) inhibiting angiogenesis, tumor growth or metastasis;
(3) treating a disease associated with CK2 activity (e.g., tumors); preferably, the tumor is a refractory cancer or a cancer insensitive to the CIGB-300 peptide, and preferably, the tumor is selected from the group consisting of prostate cancer, ovarian cancer, pancreatic cancer, colon cancer, small cell lung cancer (SCLC), non-small cell lung cancer (NSCLC), breast cancer, renal cancer, melanoma, multiple myeloma, glioblastoma, Burkitt lymphoma, leukemia, squamous cell carcinoma (e.g., laryngeal squamous cell carcinoma), adenocarcinoma (e.g., vulvar adenocarcinoma), liver cancer, and cervical cancer.

11. A method for modulating (e.g., inhibiting) CK2 activity in a cell, wherein the method comprises contacting the cell with the non-natural polypeptide according to any one of claims 1 to 7, the pharmaceutical composition according to claim 8, or the kit according to claim 9;
preferably, the cell is a tumor cell.

12. A method for inhibiting proliferation or metastasis of a tumor cell, or promoting apoptosis of the tumor cell, wherein the method comprises administering to the subject an effective amount of the non-natural polypeptide according to any one of claims 1 to 7, the pharmaceutical composition according to claim 8, or the kit according to claim 9;
preferably, the method further comprises administering to the subject an effective amount of a second therapeutic agent in combination therewith;
preferably, the second therapeutic agent is an epidermal growth factor receptor (EGFR) inhibitor, more preferably, the EGFR inhibitor is selected from the group consisting of paclitaxel, cisplatin, cytarabine (AraC), erlotinib, and nimotuzumab;
preferably, the non-natural polypeptide or the pharmaceutical composition is administered simultaneously or sequentially with the second therapeutic agent.

13. A method for inhibiting angiogenesis, or inhibiting tumor growth or metastasis, wherein the method comprises administering to the subject an effective amount of the non-natural polypeptide according to any one of claims 1 to 7, the pharmaceutical composition according to claim 8, or the kit according to claim 9;
preferably, the method further comprises administering an effective amount of a second therapeutic agent to the subject in combination therewith;
preferably, the second therapeutic agent is an epidermal growth factor receptor (EGFR) inhibitor, more preferably, the EGFR inhibitor is selected from the group consisting of paclitaxel, cisplatin, cytarabine (AraC), erlotinib, and nimotuzumab;
preferably, the non-natural polypeptide or the pharmaceutical composition is administered simultaneously or sequentially with the second therapeutic agent.

14. The use according to claim 10, or the method according to any one of claims 11 to 13, wherein the tumor cell is a cell line or a cell derived from the subject;
preferably, the tumor cell is selected from the group consisting of prostate cancer cells, ovarian cancer cells, pancreatic cancer cells, colon cancer cells, small cell lung cancer (SCLC) cells, non-small cell lung cancer (NSCLC) cells, breast cancer cells, renal cancer cells, melanoma cells, multiple myeloma cells, glioblastoma cells, Burkitt lymphoma cells, leukemia cells, squamous cell carcinoma (e.g., laryngeal squamous cell carcinoma) cells, adenocarcinoma (e.g., vulvar adenocarcinoma) cells, liver cancer cells, and cervical cancer cells.

15. A method for treating a disease associated with CK2 activity in a subject, wherein the method comprises administering to the subject a therapeutically effective amount of the non-natural polypeptide according to any one of claims 1 to 7, the pharmaceutical composition according to claim 8, or the kit according to claim 9;
preferably, the method further comprises administering to the subject a therapeutically effective amount of a second therapeutic agent in combination therewith;
preferably, the second therapeutic agent is an epidermal growth factor receptor (EGFR) inhibitor, more preferably, the EGFR inhibitor is selected from the group consisting of paclitaxel, cisplatin, cytarabine (AraC), erlotinib, and nimotuzumab;
preferably, the non-natural polypeptide or the pharmaceutical composition is administered simultaneously or sequentially with the second therapeutic agent;
preferably, the disease associated with CK2 activity is a tumor, preferably, the tumor is a refractory cancer or a cancer insensitive to the CIGB-300 peptide, and preferably, the tumor is selected from the group consisting of prostate cancer, ovarian cancer, pancreatic cancer, colon cancer, small cell lung cancer (SCLC), non-small cell lung cancer (NSCLC), breast cancer, renal cancer, melanoma, multiple myeloma, glioblastoma, Burkitt lymphoma, leukemia, squamous cell carcinoma (e.g., laryngeal squamous cell carcinoma), adenocarcinoma (e.g., vulvar adenocarcinoma), liver cancer, and cervical cancer.
